# EUROPÄISCHE PATENTSCHRIFT

(11) **EP 0 493 761 B1**
(45) Veröffentlichungstag und Bekanntmachung des Hinweises auf die Patenterteilung: **22.05.1996**
(21) Anmeldenummer: 91121930.1
(22) Anmeldetag: 20.12.1991
(51) Int. Cl.: C12P 17/18

(54) **Salinomycin-Biomasse-Aufbaugranulat, das freifliessend und staubfrei ist und uneingeschränkte Wirkstoffbioverfügbarkeit besitzt, und ein Verfahren zu dessen Herstellung**
Free-flowing and dust-free granular anabolic salinomycin biomass having unrestricted bioavailability of active agent and process for its production
Produit anabolique granulé, non poussièreux, de biomasse de salinomycine, s'écoulant librement, avec une biodisponibilité ilimitée d'agent actif et procédé pour la production d'un tel produit

(30) Priorität: 21.12.1990 DE 4041190
(43) Veröffentlichungstag der Anmeldung: 08.07.1992
(73) Patentinhaber: HOECHST AKTIENGESELLSCHAFT, D-65926 Frankfurt am Main (DE)
(72) Erfinder: Höhl, Rolf, Dr., W-6238 Hofheim am Taunus (DE)

(56) Entgegenhaltungen:
- EP-A- 0 035 125
- EP-A- 0 171 628

## Beschreibung

Aufarbeitungsverfahren von Salinomycin-Kulturbrühen sind bekannt (EP 0 035 125). In diesem Verfahren wird aus dem Feststoff der Salinomycin-Kulturbrühe ein Biomasse-Sprühpulver und anschließend mit Trägermaterial ein 6 Gew.-%iges (6 Gew.-% Salinomycingehalt), aber nicht staubfreies Aufbaugranulat hergestellt. Die sich daraus ergebenden toxikologischen und gewerbehygienischen Bedenken erfordern einen Weg zur Herstellung eines staubfreien und abriebfesten, dabei freifließenden Agglomerats (oder Mikrogranulats), das bei definierter Korngröße gute Zumischeigenschaften zu Tierfuttermitteln haben sollte und dessen Wirkstoffbioverfügbarkeit nicht eingeschränkt sein sollte. Gleichzeitig sollte ein höherprozentiges Granulat hergestellt werden.

Ein nach dem Verfahren der EP 0 035 125 oder nach dem bekannten FSD-(Fluid-Stage-Drying)-Verfahren hergestelltes Agglomerat (Aufbaugranulat) zerfällt leicht und bildet Staub, wenn der Restextraktgehalt (Restfettgehalt 3,5 Gew.-% = 17,5 Gew.-% in der Trockenmasse der Kulturbrühe) der Kulturbrühe maximal abgebaut wird.

Wäre man den bekannten Weg einer nachträglichen Granulierung mit Pressen und Mahlen gegangen, hätte es erheblicher Investitionen bedurft und das Produkt wäre entsprechend teuer geworden. Außerdem zeigt auch ein auf diesem Wege hergestelltes Versuchsprodukt immer noch nicht nicht alle gewünschten Eigenschaften, da die Qualitätsanforderungen teilweise gegenläufig sind. Bekannt ist, daß ein gut fließendes Produkt staubt, ein staubfreies Produkt aber verbackt. Außerdem besitzt ein abriebfestes Produkt nur eine eingeschränkte Bioverfügbarkeit.

Gegenstand der Erfindung ist ein Salinomycin-Biomasse-Aufbaugranulat, das freifließend und staubfrei ist, diese Staubfreiheit bei der Weiterverarbeitung auch dann behält, wenn Abriebkräfte auftreten, und dessen Wirkstoffbioverfügbarkeit nicht eingeschränkt ist.

Erfindungsgemäß ist das Granulat dadurch gekennzeichnet, daß 30 bis 40 Gewichts-% (Gew.-%) Antiback- und Fließhilfsmittel sowie 0,5 - 2 Gew.-% Celluloseether, bezogen auf das Gewicht der bei der Fermentation entstehenden Kulturbrühe, enthält, wobei das Verhältnis von Antibackmittel zu Fließhilfsmittel 3:1 bis 9:1 beträgt.

Gegenstand der Erfindung ist außerdem ein Verfahren zur Herstellung eines Salinomycin-Biomasse-Aufbaugranulats durch Sprühtrocknung einer Salinomycin-Kulturbrühe, dadurch gekennzeichnet, daß vor dem Sprühtrocknen Antibackmittel und Celluloseether zur Kulturbrühe gegeben werden und während der Sprühtrocknung das Fließhilfsmittel zugegeben wird.

Durch das erfindungsgemäße Verfahren wird in einem Schritt ein 10 - 26 Gew.-%iges, vorzugsweise 10 bis 15 Gew.-%iges Salinomycin-Biomasse-Aufbaugranulat hergestellt.

Während für das Sprühpulver nach der EP 0 035 125 ein Restfettgehalt, der niedriger als 3,5 % liegt, angestrebt wird, erweist sich für das erfindungsgemäße Aufbaugranulat-Verfahren ein Restfettgehalt von 5 - 6 %, vorzugsweise 5 - 5,6 % in der Kulturbrühe (ca. 24 - 30 %, vorzugsweise 25 - 27 %, bezogen auf die Trockenmasse), als optimal.

Der Abbau des Restfettgehaltes wird also nicht maximal durchgeführt, sondern bei einem höheren, für die obengenannten Ziele optimalen Wert abgebrochen.

In Abhängigkeit vom Salinomycin-Gehalt der Kulturbrühe werden 30 - 40 Gew.-% an Inertmaterial (Antibackmittel und Fließhilfsmittel) zugesetzt. Das Verhältnis Antibackmittel:Fließhilfsmittel beträgt 3:1 bis 9:1, vorzugsweise 7:1. Davon wird das Antibackmittel in die Kulturbrühe eingerührt. Der Rest des Inertmaterials (= Fließhilfsmittel) wird während der Trocknung in den Sprühturm eingeblasen.

Als Antibackmittel werden beispielsweise fein verteilte Calciumcarbonate und Kieselsäuren natürlicher Herkunft, beispielsweise Kreide, Diatomeenerde, Talkum oder Kaolin verwendet, als Fließhilfsmittel synthetische Kieselsäuren oder gefällte Kieselsäure, wobei die Antibackmittel sowohl allein als auch in Mischungen miteinander eingesetzt werden können.

Durch Erhöhen der Fließhilfsmittelmenge können die Fließeigenschaften signifikant verbessert werden, allerdings erhöht sich dann auch die Staubzahl, insbesondere dann, wenn bei der Weiterverarbeitung des Produktes Abriebkräfte auftreten.

Man kann dem entgegenwirken, indem man den Abbau des Restfettgehalts der Kulturbrühe nicht bis zu den maximalen Werten betreibt, sondern bei höheren Werten abbricht. Dann staubt das Produkt nicht und zeigt keinen Abrieb, aber es verklebt und verbackt wieder.

Eine Zugabe von Öl zur Staubbindung scheidet aus den gleichen Gründen aus.

Es ist bekannt, daß man Granulate durch Aufsprühen von Celluloseethern, z.B. Carboxymethylcellulose (CMC) oder ähnlichen Substanzen äußerlich härten kann. Dies hat jedoch einen negativen Einfluß auf die Bioverfügbarkeit des Wirkstoffs. Ebenso ist bekannt, daß CMC, eingerührt in eine ölige wäßrige Suspension, die Öltröpfchen feinst verteilt, was nach dem Sprühtrocknen die Staubbildung noch verstärkt.

Es wurde nun überraschend gefunden, daß man durch CMC-Zugabe zur Kulturbrühe vor der Sprühtrocknung ein Produkt erhält, das gut bioverfügbar ist, ohne daß durch die feine Verteilung der Öl- (und Salinomycin-)Tröpfchen in der Kulturbrühe die Aufbaugranulierung im Sprühtrockner behindert wird. Eingesetzt werden 0,5 - 2 Gew.-%, bezogen auf die Kulturbrühe, vorzugsweise 1 Gew.-%. Es bildet sich ein durch CMC auch innerlich gehärtetes Aufbaugranulat, das auch bei dem Auftreten von Abriebkräften nicht zur Staubbildung neigt. Das erhaltene Aufbaugranulat ist freifließend und besitzt einen Fließfaktor nach Jenike von mindestens 10.

Folgendes Korngrößenspektrum wird durch technische Parameter der Trocknungsanlage erreicht:
> 2,000 mm, 0,0 - 0,5 %, vorzugsweise 0 %
1,000 - 2,000 mm, 0,0 - 1,0 %, vorzugsweise 0 %
0,500 - 1,000 mm, 0,5 - 5 %, vorzugsweise weniger als 5 %
0,180 - 0,500 mm, 50 - 80 %, vorzugsweise 70 %
0,100 - 1,180 mm, 10 - 20 %, vorzugsweise 20 %
< 0,100 mm, 0 - 10 %, vorzugsweise weniger als 5 %.

Die folgenden Beispiele sollen die Erfindung verdeutlichen. Falls nicht anders angegeben, bedeuten %-Angaben Gewichtsprozente.

Zur Herstellung des Aufbaugranulats kann vorteilhaft ein Sprühtrockner mit integriertem Wirbelbett verwendet werden, der nach dem FSD-Verfahren arbeitet (FSD-Trockner der Fa. Niro Atomizer, Kopenhagen, Dänemark).

### Beispiel I

Salinomycin-Kulturbrühe wird in bekannter Weise fermentiert, so daß zum Fermentationsende ein Trockenmassegehalt von ca. 20 % vorliegt. Während der Fermentation wird die Ölzugabe so gesteuert, daß der extrahierbare Restfettgehalt in der Trockenmasse der fertigen Kulturbrühe bei 24 - 30 % liegt.

Danach wird der pH-Wert mit NaOH auf pH 10 eingestellt und die Kulturbrühe 2 Stunden lang auf 80°C erhitzt. Dadurch wird der Produktionsstamm restlos abgetötet.

Anschließend wird 1 Gew.-% Carboxymethylcellulose (bezogen auf die Menge Kulturbrühe) in die Kulturbrühe eingerührt. Die so behandelte Kulturbrühe wird vorzugsweise über eine Kolloidmühle in die intensiv gerührte Vorlage der Sprühtrocknungsanlage gepumpt.

Inzwischen wird der Salinomycingehalt und die Trockenmasse der Kulturbrühe sowie der Restfettgehalt in der Trockenmasse bestimmt. Bei einem Restfettgehalt von 24 - 25 % in der Trockenmasse der Kulturbrühe wird berechnet, wieviel Antibackmittel in Form von Kreide in die Trocknervorlage einzurühren ist, damit ein Salinomycin-Gehalt von 13 % im Sprühpulver erhalten wird. In diesem Fall werden 30 % Antibackmittel, bezogen auf die Menge Kulturbrühe, dazugegeben. Dabei bleibt die Brühe noch gut pumpfähig und wird auf die Düsen oder den Teller eines FSD-Sprühtrockners gepumpt. Die Brühe wird am Kopf des Trockners bei ca. 200 - 240°C Trockentemperatur eingesprüht. Das Trockengas mit weniger als 8 % Sauerstoff verläßt mit Wasser beladen den Trockner mit einer Temperatur von ca. 90°C. Das noch nicht ganz trockene Sprühpulver fällt auf das im Trocknerkonus integrierte Wirbelbett und wird dort mit ca. 85°C heißem Trockengas nachgetrocknet.

Der Feinanteil des Sprühpulvers wird mit dem Trocknungsgas ausgetragen, über Zyklone (Gerät zum Trennen feinkörniger Gemische nach der Korngröße) abgeschieden und über eine Schüttelrinne dem integrierten Fließbett wieder zugeführt.

In diesen Produktrückstrom wird so viel Kieselsäure (4 %) (Fließhilfsmittel) eindosiert, daß der Salinomycingehalt des Produktes möglichst exakt bei 12 % liegt. Während der Nachtrocknung auf dem integrierten Wirbelbett nimmt das Fließhilfsmittel an der Bildung des Aufbaugranulats teil, so daß ein freifließendes (Fließfaktor > 10), nicht zum Verbacken neigendes, nicht staubendes, abriebfestes Produkt mit guter Bioverfügbarkeit des Wirkstoffs erhalten wird. Ca. 70 % des erhaltenen Produkts hat eine Korngröße zwischen 0,180 und 0,500 mm.

### Beispiel II

Die Fermentation und die Nachbehandlung der Kulturbrühe erfolgt wie unter Beispiel I beschrieben einschließlich der Carboxymethylcellulose-Zugabe.

Die Laboranalyse ergibt einen Restfettgehalt von 30 % in der Trockenmasse der Kulturbrühe. Die Zugabe von Antibackmittel (Kreide) in die Trocknervorlage wird reduziert. Sie wird so berechnet, daß der Salinomycingehalt des Sprühpulvers noch bei 14 % liegen würde. Dagegen wird die Fließhilfsmittelzugabe (Kieselsäure) auf das Wirbelbett so weit erhöht, daß ein Endgehalt von 12 % Salinomycin möglichst exakt erhalten wird (26 % Antibackmittel, 8 % Fließhilfsmittel, bezogen auf die Kulturbrühe). Unter Beibehaltung der sonstigen Trocknungsbedingungen wird trotz des ungünstigeren Restfettgehalts der Kulturbrühe ein freifließendes (Fließfaktor > 10), nicht verbackendes, staubfreies Aufbaugranulat der gewünschten Korngröße und mit guter Bioverfügbarkeit des Wirkstoffs erhalten.

### Beispiel III

Die Aufarbeitung der Kulturbrühe erfolgt wie unter Beispiel II beschrieben unter Einsatz des Vakuumdünnschichtverdampfers. In das Konzentrat wird nur soviel von dem unter Beispiel I berechneten Antibackmittel (Kreide) eingerührt, daß die Brühe noch pump- und sprühfähig bleibt. Die Zugabe von Fließhilfsmittel (Aerosil^{(R)}, Sipernat^{(R)}) auf das Wirbelbett des FSD-Sprühtrockners wird so weit erhöht, daß ein Aufbaugranulat mit möglichst exakt 12 % Salinomycingehalt und den unter Beispiel I beschriebenen sonstigen Eigenschaften erhalten wird.

### Beispiel IV

Die Kulturbrühe wird wie unter den Beispielen I bis III beschrieben aufgearbeitet. Die physikalischen Eigenschaften werden im Laboratorium sofort getestet. Wenn die Fließeigenschaften nicht zufriedenstellen, wird die Menge Kieselsäure (Fließhilfsmittel) erhöht unter gleichzeitiger Reduktion der Antibackmittel (Kreide)-Zugabe. Wenn die Staubfreiheit nicht ausreicht, wird umgekehrt die Menge an Kreide erhöht und die Menge an Kieselsäure verringert. Die Summe an Inertmaterial ist jedoch so berechnet, daß der gewünschte Salinomycingehalt im Aufbaugranulat erhalten wird.

## Patentansprüche (Patentansprüche für folgende(n) Vertragsstaat(en): CH, DE, DK, FR, GB, IT, LI, NL)

1. Salinomycin-Biomasse-Aufbaugranulat, dadurch gekennzeichnet, daß es 30 bis 40 Gew.-% Antiback- und Fließhilfsmittel sowie 0,5 bis 2 Gew.-% Celluloseether, bezogen auf das Gewicht der bei der Fermentation entstehenden Kulturbrühe enthält, wobei das Verhältnis von Antibackmittel zu Fließhilfsmittel 3:1 bis 9:1 beträgt.

2. Salinomycin-Biomasse-Aufbaugranulat nach Anspruch 1, dadurch gekennzeichnet, daß das Aufbaugranulat einen Salinomycingehalt von 10 bis 26 Gew.-%, vorzugsweise 10 bis 15 Gew.-% und einen Fließfaktor nach Jenike von mindestens 10 hat.

3. Salinomycin-Biomasse-Aufbaugranulat nach Anspruch 1 oder 2, dadurch gekennzeichnet, daß das Aufbaugranulat folgendes Kornspektrum aufweist:
> 2,000 mm, 0,0 - 0,5 %,
1,000 - 2,000 mm, 0,0 - 1,0 %,
0,500 - 1,000 mm, 0,5 - 5 %,
0,180 - 0,500 mm, 50 - 80 %,
0,100 - 1,180 mm, 10 - 20 %,
< 0,100 mm, 0 - 10 %.

4. Verfahren zur Herstellung eines Salinomycin-Biomasse-Aufbaugranulats durch Sprühtrocknung einer Salinomycin-Kulturbrühe, dadurch gekennzeichnet, daß vor dem Sprühtrocknen Antibackmittel und Celluloseether zur Kulturbrühe gegeben werden und während der Sprühtrocknung das Fließhilfsmittel zugegeben wird.

5. Verfahren nach Anspruch 4, dadurch gekennzeichnet, daß das Fließhilfsmittel im Sprühturm in die Wirbelbettzone eindosiert wird.

6. Verfahren nach Anspruch 4 oder 5, dadurch gekennzeichnet, daß die verwendete Salinomycin-Kulturbrühe auf einen Restgehalt an extrahierbaren Fetten von 5 - 6 Gew.-% fermentiert wird und der Kulturbrühe 30 bis 40 Gew.-% eines Inertmaterials, bestehend aus einem Antibackmittel und einem Fließhilfsmittel, im Verhältnis von 3:1 bis 9:1, und ein Celluloseether in einer Menge von 0,5 bis 2 Gew.-% jeweils bezogen auf das Gesamtgewicht der Kulturbrühe, zugesetzt wird.

7. Verfahren nach einem oder mehreren der Ansprüche 4 bis 6, dadurch gekennzeichnet, daß die Kulturbrühe einen Restfettgehalt von 5 bis 5,6 Gew.-% besitzt.

8. Verfahren nach einem oder mehreren der Ansprüche 4 bis 7, dadurch gekennzeichnet, daß man als Celluloseether Carboxymethylcellulose verwendet.

9. Verfahren nach einem oder mehreren der Ansprüche 4 bis 8, dadurch gekennzeichnet, daß als Antibackmittel Kreide und als Fließhilfsmittel Kieselsäure eingesetzt werden.

## Patentansprüche (Patentansprüche für folgende(n) Vertragsstaat(en): ES)

1. Verfahren zur Herstellung eines Salinomycin-Biomasse-Aufbaugranulats durch Sprühtrocknung einer Salinomycin-Kulturbrühe, dadurch gekennzeichnet, daß vor dem Sprühtrocknen Antibackmittel und Celluloseether zur Kulturbrühe gegeben werden und während der Sprühtrocknung das Fließhilfsmittel zugegeben wird.

2. Verfahren nach Anspruch 1, dadurch gekennzeichnet, daß das Fließhilfsmittel im Sprühturm in die Wirbelbettzone eindosiert wird.

3. Verfahren nach Anspruch 1 oder 2, dadurch gekennzeichnet, daß die verwendete Salinomycin-Kulturbrühe auf einen optimalen Restgehalt an extrahierbaren Fetten von 5 bis 6 % fermentiert wird und der Kulturbrühe 30 bis 40 % eines Inertmaterials, bestehend aus einem Antibackmittel und einem Fließhilfsmittel im Verhältnis von 3:1 bis 9:1, und ein Celluloseether in einer Menge von 0,5 bis 2 Gew.-%, jeweils bezogen auf das Gesamtgewicht der Kulturbrühe, zugesetzt wird.

4. Verfahren nach einem oder mehreren der Ansprüche 1 bis 3, dadurch gekennzeichnet, daß die Kulturbrühe einen Restfettgehalt von 5 bis 5,6 Gew.-% besitzt.

5. Verfahren nach einem oder mehreren der Ansprüche 1 bis 4, dadurch gekennzeichnet, daß man als Celluloseether Carboxymethylcellulose verwendet.

6. Verfahren nach einem oder mehreren der Ansprüche 1 bis 5, dadurch gekennzeichnet, daß als Antibackmittel Kreide und als Fließhilfsmittel Kieselsäure eingesetzt werden.

## Claims (Claims for the following Contracting State(s): CH, DE, DK, FR, GB, IT, LI, NL)

1. Salinomycin biomass agglomeration granules which contain 30 to 40 % by weight of anticaking agents and flow promoters and 0.5 to 2 % by weight of cellulose ethers, based on the weight of the culture broth produced in the fermentation, where the ratio of anticaking agent to flow promoter is 3:1 to 9:1.

2. Salinomycin biomass agglomeration granules as claimed in claim 1, wherein the agglomeration granules have a salinomycin content of 10 to 26 % by weight, preferably 10 to 15 % by weight, and a Jenike flow factor of at least 10.

3. Salinomycin biomass agglomeration granules as claimed in claim 1 or 2, wherein the agglomeration granules have the following particle spectrum:
> 2.000 mm, 0.0 - 0.5 %,
1.000 - 2.000 mm, 0.0 - 1.0 %,
0.500 - 1.000 mm, 0.5 - 5 %,
0.180 - 0.500 mm, 50 - 80 %,
0.100 - 1.180 mm, 10 - 20 %,
< 0.100 mm, 0 - 10 %.

4. A process for the production of salinomycin biomass agglomeration granules by spray drying a salinomycin culture broth, which comprises adding anticaking agent and cellulose ethers to the culture broth before the spray drying, and adding the flow promoter during the spray drying.

5. The process as claimed in claim 4, wherein the flow promoter is metered into the fluidized bed zone in the spray tower.

6. The process as claimed in claim 4 or 5, wherein the salinomycin culture broth used is fermented to a residual content of 5 - 6 % by weight of extractable fats, and 30 to 40 % by weight of an inert material composed of an anticaking agent and of a flow promoter, in the ratio from 3:1 to 9:1, and a cellulose ether in an amount of from 0.5 to 2 % by weight, in each case based on the total weight of the culture broth, is added to the culture broth.

7. The process as claimed in one or more of claims 4 to 6, wherein the culture broth has a residual fat content of 5 to 5.6 % by weight.

8. The process as claimed in one or more of claims 4 to 7, wherein carboxymethylcellulose is used as cellulose ether.

9. The process as claimed in one or more of claims 4 to 8, wherein chalk is employed as anticaking agent and silica is employed as flow promoter.

## Claims (Claims for the following Contracting State(s): ES)

1. A process for the production of salinomycin biomass agglomeration granules by spray drying a salinomycin culture broth, which comprises adding anticaking agent and cellulose ethers to the culture broth before the spray drying, and adding the flow promoter during the spray drying.

2. The process as claimed in claim 1, wherein the flow promoter is metered into the fluidized bed zone in the spray tower.

3. The process as claimed in claim 1 or 2, wherein the salinomycin culture broth used is fermented to an optimum residual content of 5 to 6 % of extractable fats, and 30 to 40 % of an inert material composed of an anticaking agent and of a flow promoter in the ratio from 3:1 to 9:1, and a cellulose ether in an amount of from 0.5 to 2 % by weight, in each case based on the total weight of the culture broth, is added to the culture broth.

4. The process as claimed in one or more of claims 1 to 3, wherein the culture broth has a residual fat content of 5 to 5.6 % by weight.

5. The process as claimed in one or more of claims 1 to 4, wherein carboxymethylcellulose is used as cellulose ether.

6. The process as claimed in one or more of claims 1 to 5, wherein chalk is employed as anticaking agent and silica is employed as flow promoter.

## Revendications (Revendications pour l'(les) Etat(s) contractant(s) suivant(s): CH, DE, DK, FR, GB, IT, LI, NL)

1. Produit anabolique granulé de biomasse-salinomycine, caractérisé en ce qu'il contient de 30 à 40 % en poids d'adjuvant d'écoulement et d'antiagglutinant, ainsi que 0,5 à 2 % en poids d'éther de cellulose, par rapport au poids du bouillon de culture obtenu lors de la fermentation, le rapport de l'antiagglutinant à l'adjuvant d'écoulement allant de 3:1 à 9:1.

2. Produit anabolique granulé de biomasse-salinomycine selon la revendication 1, caractérisé en ce que le produit anabolique granulé a une teneur en salinomycine de 10 à 26 % en poids, de préférence de 10 à 15 % en poids, et un indice d'écoulement selon Jenike d'au moins 10.

3. Produit anabolique granulé de biomasse-salinomycine selon la revendication 1 ou 2, caractérisé en ce que le produit anabolique granulé présente la distribution granulométrique suivante:
> 2,000 mm, 0,0 - 0,5 %,
1,000 - 2,000 mm, 0,0 - 1,0 %,
0,500 - 1,000 mm, 0,5 - 5 %,
0,180 - 0,500 mm, 50 - 80 %,
0,100 - 1,180 mm, 10 - 20 %,
< 0,100 mm, 0 - 10 %.

4. Procédé pour l'obtention d'un produit anabolique granulé de biomasse-salinomycine par séchage par atomisation d'un bouillon de culture de salinomycine, caractérisé en ce qu'un antiagglutinant et un éther de cellulose sont ajoutés au bouillon de culture avant le séchage par atomisation et l'adjuvant d'écoulement est ajouté pendant le séchage par atomisation.

5. Procédé selon la revendication 4, caractérisé en ce que l'adjuvant d'écoulement est introduit de façon réglée dans la zone du lit fluidisé dans la tour d'atomisation.

6. Procédé selon la revendication 4 ou 5 , caractérisé en ce que le bouillon de culture de salinomycine utilisé est fermenté jusqu'à une teneur résiduelle optimale en matières grasses extractibles de 5 à 6 % et on ajoute au bouillon de culture 30 à 40 % d'une substance inerte, consistant en un antiagglutinant et un adjuvant d'écoulement, en un rapport allant de 3:1 à 9:1, et un éther de cellulose en une proportion de 0,5 à 2 % en poids, dans chaque cas par rapport au poids total du bouillon de culture.

7. Procédé selon une ou plusieurs des revendications 4 à 6, caractérisé en ce que le bouillon de culture a une teneur résiduelle en matière grasse de 5 à 5,6 % en poids.

8. Procédé selon une ou plusieurs des revendications 4 à 7, caractérisé en ce que, comme éther de cellulose, on utilise la carboxyméthylcellulose.

9. Procédé selon une ou plusieurs des revendications 4 à 8, caractérisé en ce que, comme antiagglutinant, on utilise la craie et, comme adjuvant d'écoulement, on utilise la silice.

## Revendications (Revendications pour l'(les) Etat(s) contractant(s) suivant(s): ES)

1. Procédé pour l'obtention d'un produit anabolique granulé de biomasse-salinomycine par séchage par atomisation d'un bouillon de culture de salinomycine, caractérisé en ce qu'un antiagglutinant et un éther de cellulose sont ajoutés au bouillon de culture avant le séchage par atomisation et l'adjuvant d'écoulement est ajouté pendant le séchage par atomisation.

2. Procédé selon la revendication 1, caractérisé en ce que l'adjuvant d'écoulement est introduit de façon réglée dans la zone du lit fluidisé dans la tour d'atomisation.

3. Procédé selon la revendication 1 ou 2, caractérisé en ce que le bouillon de culture de salinomycine utilisé est fermenté jusqu'à une teneur résiduelle optimale en matières grasses extractibles de 5 à 6 % et on ajoute au bouillon de culture 30 à 40 % d'une substance inerte, consistant en un antiagglutinant et un adjuvant d'écoulement, en un rapport allant de 3:1 à 9:1, et un éther de cellulose en une proportion de 0,5 à 2 % en poids, dans chaque cas par rapport au poids total du bouillon de culture.

4. Procédé selon une ou plusieurs des revendications 1 à 3, caractérisé en ce que le bouillon de culture a une teneur résiduelle en matière grasse de 5 à 5,6 % en poids.

5. Procédé selon une ou plusieurs des revendications 1 à 4, caractérisé en ce que, comme éther de cellulose, on utilise la carboxyméthylcellulose.

6. Procédé selon une ou plusieurs des revendications 1 à 5, caractérisé en ce que, comme antiagglutinant, on utilise la craie et, comme adjuvant d'écoulement, on utilise la silice.
